# EUROPEAN PATENT APPLICATION

(11) **EP 4 684 763 A1**
(43) Date of publication of application: **28.01.2026**
(21) Application number: 25191998.1
(22) Date of filing: 25.07.2025
(51) Int. Cl.: A61F 2/44

(54) **CORONAL PLANE LATERAL EXPANDABLE IMPLANTS**

(30) Priority: 26.07.2024 US 202418785005
(71) Applicant: Globus Medical, Inc., Audubon, PA 19403 (US)
(72) Inventor: MCQUAID, Andrew, West Chester, 19380 (US); LEVINS, Phillip, Conshohocken, 19428 (US); MICCIO, Mark, West Collingswood Heights, 08059 (US)
(74) Representative: Morabito, Sara

(57) **Abstract**

Expandable intervertebral fusion implants, system, and methods for coronal correction. The implant may include top and bottom endplates configured to engage adjacent vertebrae, a dual actuator assembly including a rotatable drive screw actuator and a rotatable actuator nut concentric to the drive screw actuator, and driving ramps positioned along the shaft of the drive screw actuator and engaged with the top and bottom endplates via complementary ramped surfaces. When inserted in a disc space, the implant has an ipsilateral side and a contralateral side, and rotation of the drive screw actuator and/or the actuator nut causes movement of one or more of the driving ramps, thereby causing independent expansion in height of the contralateral and/or ipsilateral sides of the implant to correct the coronal deformity.

## Description

### FIELD OF THE INVENTION

The present disclosure generally relates to devices and methods for promoting an intervertebral fusion, and more particularly relates to expandable fusion devices capable of being inserted between adjacent vertebrae for disc height restoration and coronal correction.

### BACKGROUND OF THE INVENTION

A common procedure for handling pain associated with intervertebral discs that have become degenerated due to various factors, such as trauma or aging, is the use of intervertebral fusion devices for fusing one or more adjacent vertebral bodies. Generally, to fuse the adjacent vertebral bodies, the intervertebral disc is first partially or fully removed. An intervertebral fusion device is then inserted between neighboring vertebrae to maintain normal disc spacing and restore spinal stability, thereby facilitating an intervertebral fusion.

Patients with thoracolumbar spinal deformities may experience vertebral wedging or scoliosis of the spine, which creates an unwanted bend in the coronal plane, in addition to nerve root or spinal cord compression causing pain. To address this, static implants with a coronal taper may be used, but do not allow for precise adjustments needed for patients with a coronal imbalance. Expandable implants may be expanded in height, but do not offer asymmetric coronal expansion to address coronal corrections and vertebral wedging. As such, there exists a need for fusion devices capable of being installed inside an intervertebral disc space at a minimum height and expanded in height with coronal adjustments to correct coronal deformities.

### SUMMARY OF THE INVENTION

To meet this and other needs, devices, systems, and methods for performing intervertebral fusion, coronal correction, and spine stabilization are provided. In particular, expandable coronal implants, for example, for lateral or anterior spinal surgery may be used to treat a variety of patient indications. The expandable coronal implants are configured for height expansion for disc height restoration as well as asymmetric coronal expansion to address vertebral wedging and for coronal correction. The implant may be configured for contralateral or ipsilateral coronal angulation to address asymmetries and misalignments in the coronal (frontal) plane. By angulating the implant ipsilaterally or contralaterally, corrective forces may be applied directly to the curvature, thereby realigning the spine to improve biomechanical balance and stability.

According to one embodiment, an expandable coronal implant includes a top endplate and a bottom endplate configured to engage adjacent vertebrae, a dual actuator assembly including a rotatable drive screw actuator having a shaft and a rotatable actuator nut concentric to the drive screw actuator, and a plurality of driving ramps including a front ramp, a mid-ramp, and a rear ramp positioned along the shaft of the drive screw actuator and engaged with the top and bottom endplates via complementary ramped surfaces. When inserted in a disc space, the implant has an ipsilateral side and a contralateral side, and rotation of the drive screw actuator and/or the actuator nut causes movement of one or more of the driving ramps, thereby causing independent expansion in height of the contralateral and/or ipsilateral sides of the implant to correct a coronal deformity.

The expandable implant may include one or more of the following features. The dual actuator assembly may be laterally offset relative to a central longitudinal axis of the implant, and the front ramp, mid-ramp, and rear ramp may define through bores along the offset axis to receive the shaft of the drive screw actuator. The rear ramp may define female ramped grooves forming a male triangular area with a vertex pointing toward the front of the implant, the mid-ramp may define female ramped grooves forming a male triangular area with a vertex pointing toward the rear of the implant, and the front ramp may define female ramped grooves forming a male triangular area with a vertex pointing toward the front of the implant. The shaft of the drive screw actuator may include a first threaded portion, a second threaded portion, a first non-threaded portion separating the first threaded portion from the second threaded portion, and a second non-threaded portion toward a distal end of the shaft. The rear ramp may be positioned on the actuator nut, which may be positioned on the first threaded portion of the drive screw actuator, the mid-ramp may be positioned on the second threaded portion of the drive screw actuator, and the front ramp may be positioned on the second non-threaded portion of the drive screw actuator. The actuator nut may include a cylindrical body having an enlarged head defining an instrument recess and a neck with a reduced diameter defining an internal threaded portion, which engages with the first threaded portion of the shaft of the drive screw actuator. The actuator nut may be secured to the rear ramp with a first retaining clip fit into an annular groove along an outside of the actuator nut and a corresponding interior annular groove along an interior of the rear ramp. The actuator nut may be further secured to the rear ramp with a second retaining clip fit beneath the first retaining clip around the head of the actuator nut and resting in the interior annual groove along the interior of the rear ramp.

According to one embodiment, an expandable coronal implant includes a top endplate and a bottom endplate configured to engage adjacent vertebrae, wherein the top and bottom endplates are angled to provide an initial reverse taper configured to match an angle of a disc space upon insertion, a dual actuator assembly including a rotatable drive screw actuator having a shaft and a rotatable actuator nut concentric to the drive screw actuator, and a plurality of driving ramps including a front ramp, a mid-ramp, and a rear ramp positioned along the shaft of the drive screw actuator and engaged with the top and bottom endplates via complementary ramped surfaces. After insertion into the disc space, the implant is expanded by rotating the drive screw actuator and/or the actuator nut to cause movement of one or more of the driving ramps, thereby causing the endplates to expand to provide a neutral position or angle opposite to the initial reverse taper to correct a coronal deformity. The implant may have an ipsilateral side and a contralateral side in the disc space, and the initial reverse taper may have an asymmetric taper such that the contralateral side is greater in height than the ipsilateral side. The implant may be expandable to tilt the top and bottom endplates to the angle opposite to the initial reverse taper such that the ipsilateral side is greater in height than the contralateral side to correct the coronal deformity. The implant may be expandable such that the top and bottom endplates are parallel to one another in the neutral position.

According to one embodiment, a method of correcting a coronal deformity may include one or more of the following steps in any suitable order: (a) inserting an implant through a lateral approach into a disc space of a spine having a coronal deformity, the implant having top and bottom endplates, a dual actuator assembly including a rotatable drive screw actuator having a shaft and a rotatable actuator nut concentric to the drive screw actuator, and a plurality of driving ramps including a front ramp, a mid-ramp, and a rear ramp positioned along the shaft of the drive screw actuator and engaged with the top and bottom endplates via complementary ramped surfaces; (b) expanding the implant to a height to restore disc spacing; and (c) independently adjusting the height of contralateral and ipsilateral sides of the implant to provide a desired coronal angle. The drive screw actuator and actuator nut may be rotated simultaneously to move the rear and mid-ramp together in parallel, thereby moving the top and bottom endplates in parallel. Only the actuator nut may be rotated such that the rear ramp is translated forward to increase the ipsilateral side of the implant. Only the drive screw actuator may be rotated such that the mid-ramp is drawn backward to increase the contralateral side of the implant. The implant may include an initial reverse taper to match an obliquity of the disc space upon insertion. After insertion, the top and bottom endplates may be tilted to a neutral position to achieve optimal spinal alignment or the top and bottom endplates may be tilted to angle the top and bottom endplates opposite to the initial reverse taper, thereby providing an adjusted angular position for optimal correction. The method may also include correcting any compensatory curves above the coronal deformity because such a compensation is no longer necessary.

According to yet another embodiment, a kit may include a plurality of implants of different sizes and configurations. The kit may further include one or more devices suitable for installing and/or removing the implants and systems described herein, such as insertion devices or drivers; one or more removal devices; and other tools and devices, which may be suitable for surgery.

### BRIEF DESCRIPTION OF THE DRAWINGS

The present embodiments will become more fully understood from the detailed description and the accompanying drawings, wherein:
FIG. 1 illustrates the lumbar spine with an implant inserted laterally for height expansion and coronal tilt;
FIGS. 2A-2C depicts a coronal target, sweeping, and dual expansion approaches, respectively, for correcting a coronal spinal deformity;
FIG. 3 shows an expandable coronal implant with dual expansion according to one embodiment;
FIGS. 4A-4B show exploded views of the expandable coronal implant of FIG. 3;
FIGS. 5A-5C show perspective views of the rear, mid, and front driving ramps, respectively, for the expandable coronal implant;
FIGS. 6A-6B show perspective views of the actuator nut and actuator screw, respectively, for the expandable coronal implant;
FIGS. 7A-7C show cross-sectional views of the expandable coronal implant with parallel, contralateral, and ipsilateral angle bias, respectively;
FIGS. 8A-8B show alternative embodiments for angulation of the endplate using a pin in slot configuration;
FIGS. 9A-9B show an alternative expandable coronal implant having two internal screws for independent raising of the near or far side of the implant; and
FIGS. 10A-10B show a coronal target expandable implant according to another embodiment.

### DETAILED DESCRIPTION

Embodiments of the disclosure are generally directed to devices, systems, and methods for intervertebral fusion, coronal correction, and spine stabilization. Specifically, expandable coronal implants are configured to expand in height for disc height restoration and to a given coronal angle for coronal correction and vertebral wedging. The expandable coronal implant may be implanted laterally or anteriorly, for example, using an inserter instrument and expanded to a given coronal angle and height. The expandable implant may provide for contralateral or ipsilateral coronal angulation. Contralateral angulation involves adjusting the implant on the side opposite to the direction of the deformity. For instance, if the spine curves to the left, the implant may be angulated to the right to counteract this deviation. In contrast, ipsilateral angulation adjusts the implant on the same side as the deformity. This may similarly involve angulating the implant to correct the deformity or making adjustments to support structural integrity and relieve pressure on the spinal column. The ability to adjust angulation on either side of the spine provides surgeons with greater flexibility to tailor the treatment to the specific characteristics of the coronal spinal deformity.

A spinal fusion is typically employed to eliminate pain caused by motion of degenerated disc material or deformity. Upon successful fusion, a fusion device becomes permanently fixed within the intervertebral disc space. The expandable fusion device may be positioned between adjacent vertebral bodies in a collapsed position. In this case, the expandable coronal fusion implant is configured to expand in height with asymmetric coronal expansion to address coronal curvature. Whether the curvature is due to scoliosis, kyphosis, or other condition, the adjustable angulation of the implant allows for customized intervention. The implant engages the endplates of the adjacent vertebral bodies and, in the installed position, maintains desired intervertebral disc spacing, adjusts coronal angle, and restores spinal stability, thereby correcting the coronal deformity and facilitating the intervertebral fusion.

Minimally invasive surgery (MIS) may be used to preserve muscular anatomy by only causing disruption where necessary. The benefit of the MIS surgical approach is that it can reduce post-operative pain and improve recovery time for patients. In one embodiment, the expandable fusion device can be configured to be placed down an endoscopic tube and into the surgical target site. By way of example, the surgical site may be an intervertebral disc space situated between two adjacent vertebrae. The implant may be installed laterally to allow for contralateral and ipsilateral angulation for correction of coronal deformities. With precise angulation adjustments, the implant allows for focused and targeted correction, which can reduce the need for additional interventions, leading to quicker recoveries and reduced complications. Although particularly suited for use in lateral fusions, it will be readily appreciated by those skilled in the art that the implant may be employed in any number of suitable orthopedic approaches and procedures, including but not limited to, anterior, posterior, transforaminal, anterolateral, or posterolateral approaches to the lumbar spine, cervical spine, or thoracic spine, as well as any non-spine application, such as treatment of bone fractures and the like.

Components of all of the devices disclosed herein may be manufactured of any suitable materials including metals (e.g., titanium), metal alloys (e.g., stainless steel, cobalt-chromium, and titanium alloys), ceramics, plastics, plastic composites, or polymeric materials (e.g., polyether ether ketone (PEEK), polyphenylene sulfone (PPSU), polysulfone (PSU), polycarbonate (PC), polyetherimide (PEI), polypropylene (PP), polyacetals, or mixtures or co-polymers thereof), and/or combinations thereof. In some embodiments, the devices may include radiolucent and/or radiopaque materials. The components can also be machined and/or manufactured using any suitable techniques (e.g., 3D printing).

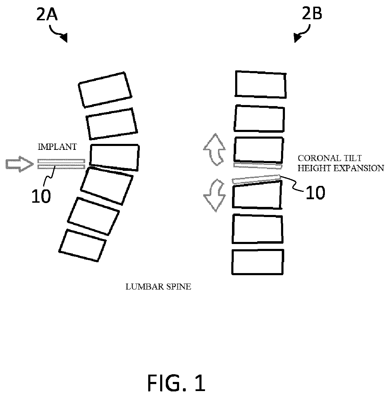

Turning now to the drawing, where like reference numerals may refer to like elements, FIG. 1 illustrates a portion of the lumbar spine, for example, lumbar vertebrae L1-L5 and sacrum S1. The left side shows the lumbar spine 2A with a lumbar imbalance and deformity causing an extreme curvature to the spine (e.g., curving to the left) and the right side shows the lumbar spine 2B corrected with an implant 10 inserted and adjusted with coronal tilt between two vertebrae. As shown, the implant 10 may be installed laterally at an apex of the spinal curvature or at another suitable spinal level. In this example, the ipsilateral height of the implant is increased to impart a coronal tilt to straighten the spine. Using the implant 10, the coronal angle can be manipulated with height expansion to correct the deformity at the apex of the concave curve. Additionally, any compensatory curves above the deformity may be corrected because the compensation is no longer necessary.

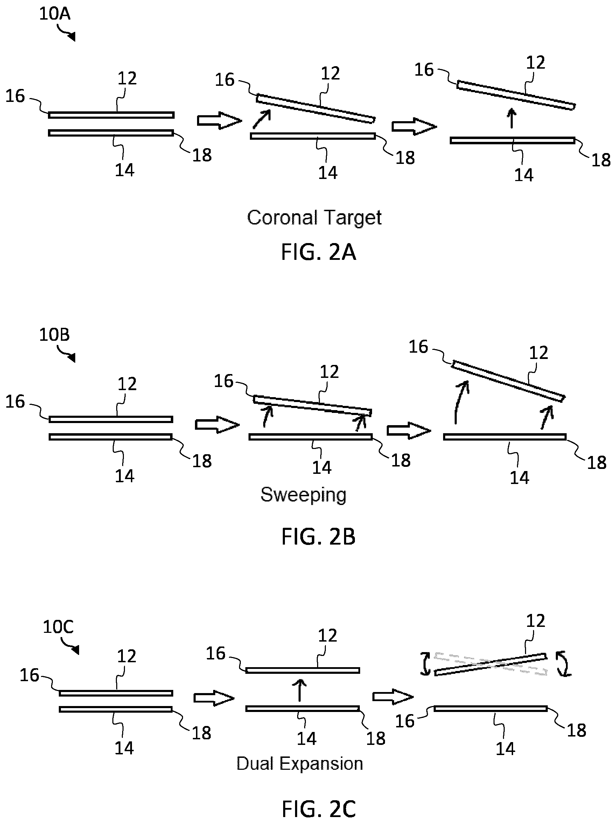

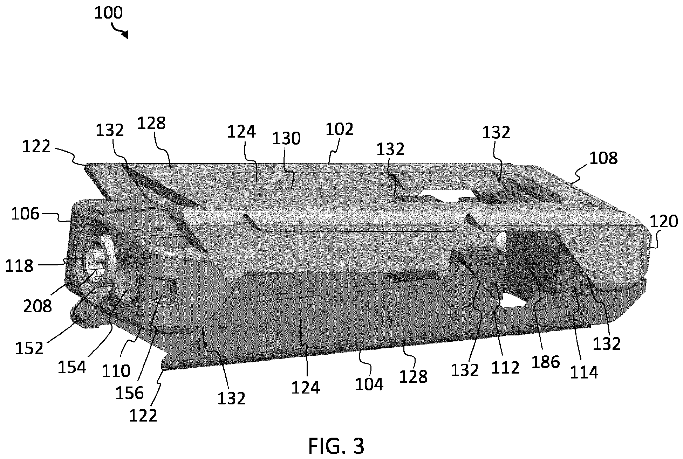

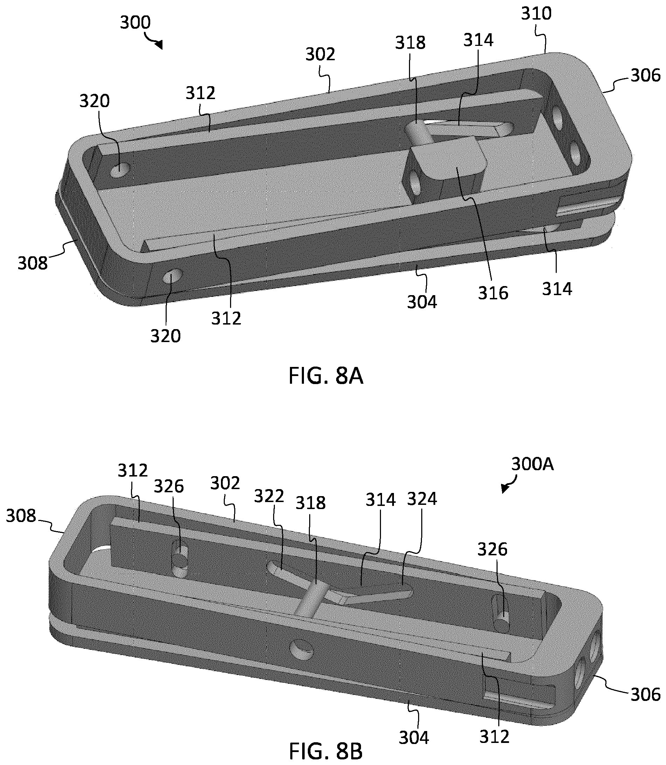

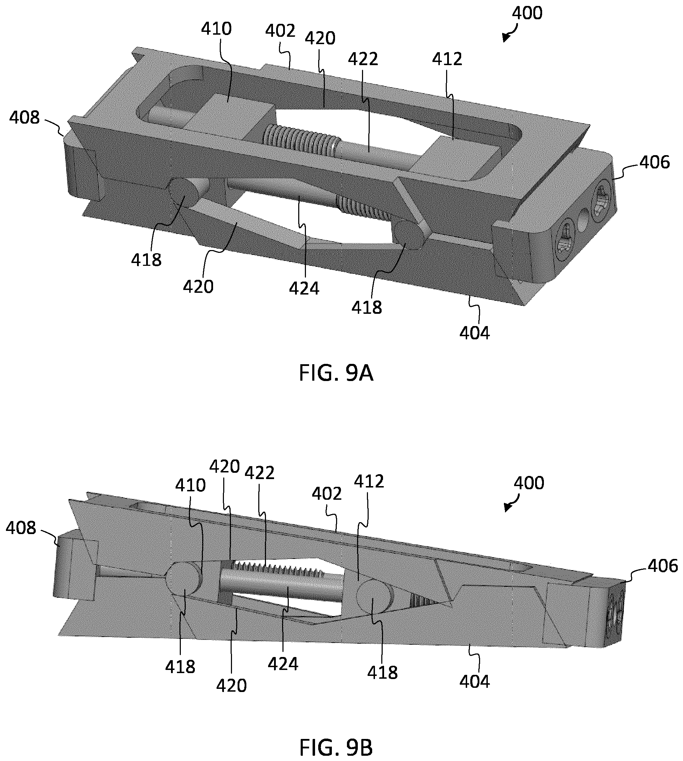

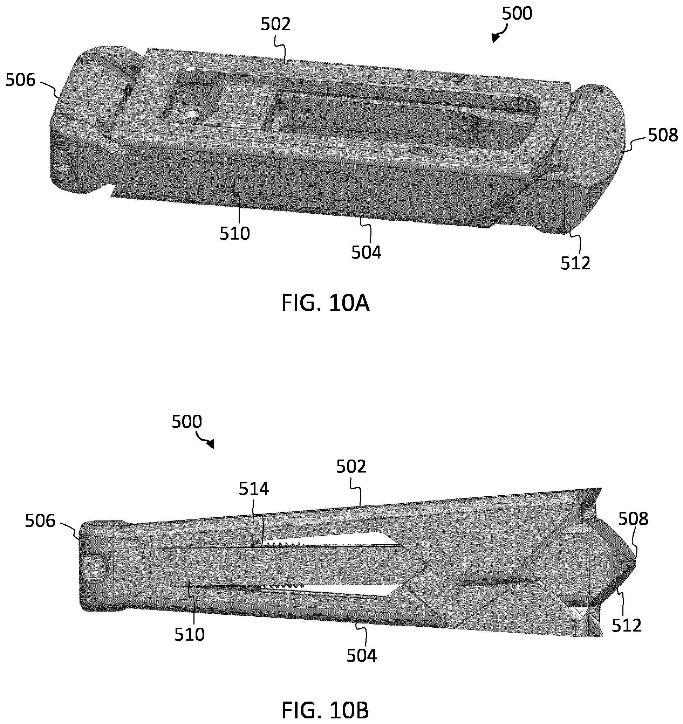

Turning now to FIGS. 2A-2C, several different approaches may be used to correct the deformity with implant 10 having expandable top and bottom endplates 12, 14, with an ipsilateral side 16 (e.g., rear of implant) and a contralateral side 18 (e.g., front of implant). As shown in FIG. 2A, a coronal target approach provides coronal tilt followed by height expansion of the implant 10 to correct the deformity. The implant 10A is positioned in a collapsed state, the coronal tilt is introduced, for example, on the ipsilateral side 16 of the implant 10A, and then the endplate(s) 12, 14 are expanded in height. As described in more detail below, FIGS. 10A-10B depicts one example of a coronal target implant 500. In FIG. 2B, the implant 10B may be configured to expand in height asymmetrically on the ipsilateral and contralateral sides 16, 18 of the disc space, creating an increasing coronal angle throughout the expansion range. The implant 10B is positioned in a collapsed state, the coronal tilt is introduced together on the ipsilateral and contralateral sides 16, 18, and the endplate(s) 12, 14 are expanded in height, thereby increasing the coronal angle throughout the range of expansion. The mechanism may expand asymmetrically in either direction relative to the convexity of the spinal deformity and approach direction, thereby providing a sweeping approach. In FIG. 2C, the implant 10C may be independently controlled with a dual expansion mechanism, which allows for independent ipsilateral and contralateral height expansion. The implant 10C is positioned in a collapsed state, the endplate(s) 12, 14 are expanded in height, and the coronal tilt is introduced independently on the ipsilateral and/or contralateral sides 16, 18 to the final desired coronal angle. As described in more detail below, FIGS. 3-7C depict an independently controlled dual expansion implant 100 with two concentric actuators, FIGS. 8A-8B show examples of dual expansion implants 300, 300A with pin and slot actuators, and FIGS. 9A-9B show a dual expansion implant 400 with a pin and ramp configuration having two separate internal screws. It will be appreciated that any suitable mechanism for height and coronal angle adjustments may be used for dual expansion to thereby provide the desired coronal correction.

The top and bottom endplates 12, 14 may be initially aligned in parallel, with a coronal taper, or with a reverse taper. In one embodiment, the top and/or bottom endplates 12, 14 of the implant 10 may be angled to provide an initial reverse taper configured to match an angle of the disc space upon insertion. After insertion, one or both of the endplates 12, 14 may be expanded to tilt the endplates 12, 14 to a neutral position or to angle the endplate 12, 14 opposite to the initial reverse taper, thereby providing an adjusted angular position for optimal coronal correction. The top and bottom endplates 12, 14 may be designed with a reverse taper intended to match or conform to the tilted or uneven disc space seen in spinal deformities, like scoliosis. For example, the initial reverse taper may have an asymmetric taper such that one side (e.g., contralateral 18) is intentionally made higher than the other (e.g., ipsilateral 16) or vice versa. The reverse taper may help in accommodating the angled disc space more naturally and inserting the implant 10 with greater ease. After insertion, the implant 10 may be expanded to tilt the top and bottom endplates 12, 14 to a neutral position (e.g., in parallel) or to an angle opposite to the initial reverse taper. For example, the ipsilateral side 16 may be expanded greater in height than the contralateral side 18 to correct the coronal deformity (or vice versa depending on the slope of the initial reverse taper). By expanding the implant 10 asymmetrically in the opposite direction as the initial installation, the vertebral endplates can shift to provide an angle opposite to the initial obliquity, thereby helping to correct coronal imbalance and achieve better spinal alignment.

With further reference to FIGS. 3-7C, the expandable coronal implant 100 may include an independently controlled dual expansion mechanism, which allows for independent ipsilateral and contralateral height expansion. FIG. 3 shows the expandable fusion implant 100 partially expanded with near side coronal angle. The expandable implant 100 includes top and bottom endplates 102, 104, which are configured to engage with adjacent vertebrae. The implant 100 extends along a central longitudinal axis between rear end 106 (or ipsilateral side) configured to connect with an insertion instrument and front end 108 (or contralateral side) configured to be inserted first into the disc space. It should be understood that reference to the rear and front ends 106, 108 are described with respect to the direction of placement into an intervertebral disc space with the front 108 of the expandable fusion implant 100 placed into the disc space first (e.g., the contralateral side), followed by the rear 106 of the expandable fusion implant 100 (e.g., the ipsilateral side). These and other directional terms may be used herein for descriptive purposes and do not limit the orientation(s) in which the devices may be used.

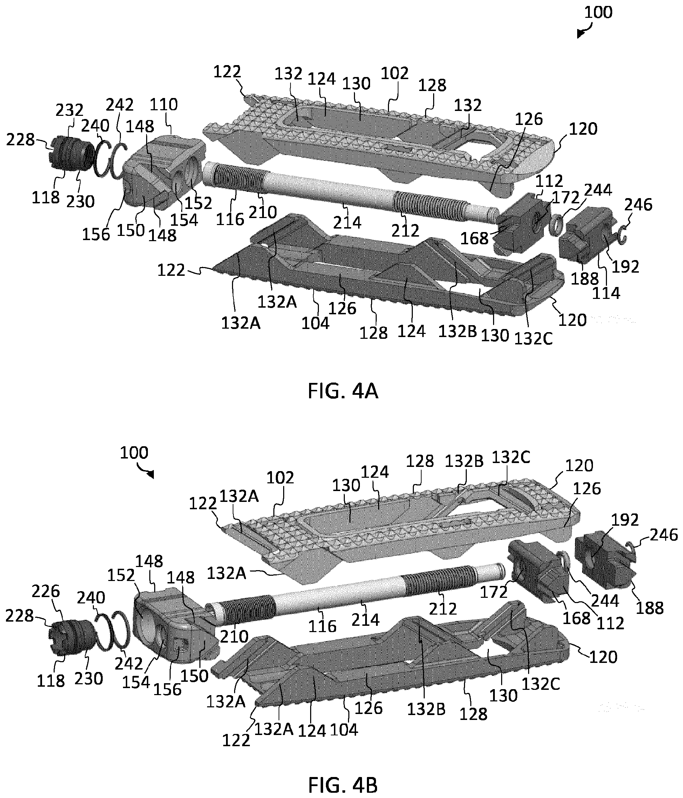

With emphasis on FIGS. 4A-4B, exploded views of the expandable coronal implant 100 are shown. The expandable implant 100 includes upper or top endplate 102, lower or bottom endplate 104, and a plurality of internal driving ramps 110, 112, 114 driven by dual actuators 116, 118 to expand the top and bottom endplates 102, 104. The internal driving ramps include a rear ramp 110, a mid-ramp 112, and a front ramp 114, which engage with the upper and lower endplates 102, 104 to expand the implant 100. The dual actuator assembly includes a central drive screw actuator 116 and an actuator nut 118, which control the driving ramps 110, 112, 114, by independently turning one or both of the actuators 116, 118. By rotating one actuator 116, 118 and holding the other in place, either the ipsilateral or contralateral side 106, 108 of the implant 100 can be lifted to the desired coronal angle. This angle may match the obliquity of the vertebral endplates, or can be used to correct the deformity, after achieving height correction.

The top endplate 102 is described in further detail herein and the description applies equally to bottom endplate 104, which may include similar features to the top endplate 102 (e.g., a mirror image). The endplate 102, 104 may include a front end 120, an opposite rear end 122, and a pair of sidewalls 124 connecting the front end 120 to the rear end 122. The endplate 102, 104 may form a generally quadrilateral shape, such as a rectangular shape with long sidewalls 124, or any other suitable shape. The front end 120 may include a smooth tapered nose to facilitate insertion into the disc space. The endplate 102, 104 includes an inner face 126 and an opposite outer face 128 configured to contact the adjacent vertebra. The outer face 128 may include a plurality of teeth or other friction increasing elements, such as ridges, roughened surfaces, keels, gripping or purchasing projections configured to retain the implant 100 in the disc space. The endplate 102, 104 may define one or more windows 130 extending between the outer and inner faces 126, 128. The windows 130 may be configured to receive bone graft or similar bone growth inducing material, which may be introduced within and/or around the device 100 to further promote and facilitate bone growth.

The sidewalls 124 define a plurality of ramped surfaces 132, which are configured to mate with the corresponding driving ramps 110, 112, 114. The ramped surfaces 132 may include angled surfaces, tracks, grooves, channels, or other mating areas configured to create a slidable interface between the driving ramps 110, 112, 114 and the endplates 102, 104. The ramped surfaces 132 may be angled, diagonal, inclined, or slanted to provide sliding engagement with the corresponding driving ramps 110, 112, 114. The ramped surfaces 132 may define female channels or inner slots, which form male projections or overhangs that are configured to be received in the female counterparts of the driving ramps 110, 112, 114. In particular, a pair of rear-most ramped surfaces 132A defining inner-facing slots with projections are configured to engage the rear driving ramp 110. When approached from the rear end 106, the slope of the angled ramp surfaces 132A may be descending. A pair of central ramped surfaces 132B defining inner-facing slots with projections are configured to engage the central driving ramp 112. When approached from the rear end 106, the slope of the angled surfaces 132B may be ascending. A pair of front-most ramped surfaces 132C defining inner-facing slots with male projections are configured to engage the front ramp 114. When approached from the rear end 106, the slope of the front-most angled surfaces 132C may be descending. The slope of each ramped surface 132 may be the same or different to achieve the desired rate and amount of expansion.

The bottom endplate 104 may be similar to endplate 102 (e.g., a mirror image). In this sense, the slope of rear-most ramped surfaces 132A, when approached from the rear end 106, may be ascending. The slope of central ramped surfaces 132B, when approached from the rear end 106, may be descending. The slope of the front-most ramped surfaces 132C, when approached from the rear end 106, may be ascending. Although the ramped surfaces 132 are shown in a given configuration, it will be appreciated that the ramped surfaces may be sloped, slanted, curved, or otherwise configured in any manner to provide the desired type of expansion, the female/male configurations may be reversed, or other suitable ramp interactions, sliding features, or mating components may be used to provide expansion of the top and bottom endplates 102, 104.

The actuation mechanism may include a plurality of internal driving ramps 110, 112, 114 driven by dual actuators 116, 118 to expand the top and bottom endplates 102, 104. The dual actuators 116, 118 may be aligned along a longitudinal axis, which is laterally offset toward one sidewall 124 within the internal driving ramps 110, 112, 114. The offset axis may be parallel and laterally offset to one side of the central longitudinal axis of the implant 100. The dual actuators 116, 118 may be concentric to one another along the offset axis. With the concentric configuration, each actuator 116, 118 may be controlled independently without interfering with the movement of the other, which allows for precise control of the internal driving ramps 110, 112, 114 and corresponding expansion of the endplates 102, 104.

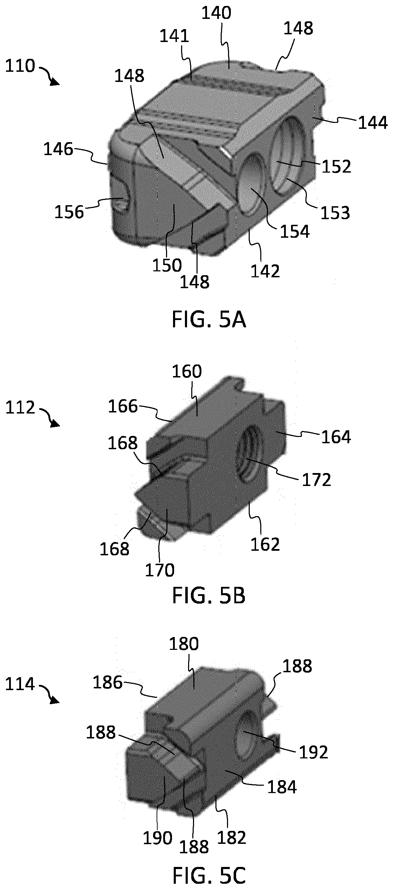

As best seen in FIG. 5A, the rear driving ramp 110 may include a block-like body located toward the proximal or rear end 106 of the implant 100. The rear driving ramp 110 may include an upper surface 140 configured to mate with the top endplate 102 and a lower surface 142 configured to mate with the bottom endplate 104 when in a collapsed configuration. The rear driving ramp 110 includes a front facing surface 144 and an opposite rear facing surface 146 configured to interface with an insertion instrument. The upper and/or lower surfaces 140, 142 may define one or more channels grooves 141 between the front and rear faces 144, 146. For example, parallel grooves 141 may be used to facilitate linear translation of the rear driving ramp 110.

The opposite side surfaces of the rear driving ramp 110 define ramped surfaces 148 configured and dimensioned to interface with the corresponding rear-most ramped surfaces 132A of the top and bottom endplates 102, 104, respectively. The ramped surfaces 148 may be angled continuous surfaces with a given angle of slope, which engage with the corresponding ramped surfaces 132A of the endplates 102, 104. For example, the ramped surfaces 148 may define female grooves or channels forming male projections, which engage with corresponding ramps 132A in the endplates 102, 104. For example, the male projections may include a triangular area 150 defined between two converging angled grooves. The triangular area 150 may extend laterally from both sides of the driving ramp 110. On each side, one upper ramped groove 148 above the triangular area 150 may have a decreasing slope and one lower ramped groove 148 below the triangular area 150 may have an increasing slope when approached from the rear end 106 of the implant 100, thereby forming a vertex of the triangular area 150 pointing toward the front 144 of the rear driving ramp 110. The ramped surfaces 148 of the rear driving ramp 110 complement the ramped surfaces 132A of the endplates 102, 104, for example, with a sliding dovetail type configuration. It will be appreciated that the female/male configurations may be reversed or may include other suitable ramp interactions, sliding features, or mating components to provide expansion. When the rear driving ramp 110 is translated toward the front or rear ends 106, 108 of the implant 100, the ramped surfaces 148 of the driving ramp 110 slide against the ramped surfaces 132A of the endplates 102, 104, thereby increasing or decreasing the height of the rear end 106 (e.g., ipsilateral side) of the implant 100.

The rear driving ramp 110 defines a first internal bore 152 between front and rear faces 144, 146 of the driving ramp 110, which is configured for receiving the concentric actuators 116, 118. The axis of the bore 152 may be aligned with the laterally offset longitudinal axis of the dual actuator drive assembly when assembled. The bore 152 may define a pronounced interior rim 153 near the front face 144, which helps to secure the actuator nut 118 therein. The rear driving ramp 110 may define a second internal bore 154 between the front and rear faces 144, 146 of the rear driving ramp 110, which may be configured for coupling to an inserter instrument and/or for receipt of graft material, for example. The second bore 154 may have a diameter smaller than the first bore 152. The second internal bore 154 may be parallel to and laterally offset to the opposite sidewall 124 and opposite to the first internal bore 152. The rear driving ramp 110 may define one or more instrument recesses or indentations 156 configured for connecting an instrument, such as an insertion instrument or implant inserter. For example, a pair of indentations 156 may be provided on opposite sides of the rear driving ramp 110 to aid in implantation and/or expansion.

As best seen in FIG. 5B, the mid-ramp 112 may include a block-like body located centrally and toward the distal or front end 108 of the implant 100. The mid-ramp 112 may have a plus-shaped cross section with an upper surface 160, a lower surface 162, a front face 164, and a rear face 166. The upper and lower surfaces 160, 162 may be flat or substantially planar with sharp corners connected to the front face 164. The sides of the ramp 112 may include wings or protrusions, which define the ramped surfaces 168 that complement the corresponding ramped surfaces 132B of the top and bottom endplates 102, 104. The ramped surfaces 168 may define female grooves or channels forming male projections, which engage with the corresponding ramped surfaces 132B in the endplates 102, 104. For example, the male projections may include a triangular area 170 defined between two converging angled grooves. On each side, one upper ramped groove 168 above the triangular area 170 may have an increasing slope and one lower ramped groove 168 below the triangular area 170 may have a decreasing slope when approached from the rear end 106 of the implant 100, thereby forming a vertex of the triangular area 170 pointing toward the rear 166 of the mid-ramp 112. The ramped surfaces 168 of the mid-ramp 112 complement the ramped surfaces 132B of the endplates 102, 104, for example, with a sliding dovetail type configuration. It will be appreciated that the female/male configurations may be reversed or may include other suitable ramp interactions, sliding features, or mating components to provide expansion. When the central driving ramp 112 is translated, the ramped surfaces 168 of the driving ramp 112 slide against the ramped surfaces 132B of the endplates 102, 104, thereby increasing or decreasing the height of the front end 108 of the implant 100.

The mid-ramp 112 defines a through bore 172 between front and rear faces 164, 166 of the driving ramp 112, which is configured for receiving the drive screw actuator 116 therethrough. The through bore 172 may be internally threaded to interface with a threaded portion 212 of the drive screw actuator 116. The threaded through bore 172 is laterally offset through the mid-ramp 112 such that the axis of the threaded through bore 172 aligns with the lateral offset axis of the drive assembly when assembled.

As best seen in FIG. 5C, the front ramp 114 may include a block-like body located toward the distal or front end 108 of the implant 100. The front ramp 114 includes an upper surface 180, a lower surface 182, a front face 184, and a rear face 186. The upper and lower surfaces 180, 182 may be flat or substantially planar with rounded corners connected to the front face 184. The sides of the ramp 114 may include wings or protrusions, which define the ramped surfaces 188 that are configured to mate with the corresponding ramped surfaces 132C of the top and bottom endplates 102, 104. The ramped surfaces 188 may define female grooves or channels forming male projections, which engage with corresponding ramps 132C in the endplates 102, 104. For example, the male projections may include a triangular area 190 defined between two converging angled grooves. On each side, one upper ramped groove 188 above the triangular area 190 may have a decreasing slope and one lower ramped groove 188 below the triangular area 190 may have an increasing slope when approached from the rear end 106 of the implant 100, thereby forming a vertex of the triangular area 190 pointing toward the front 184 of the front ramp 114. The ramped surfaces 188 of the front ramp 114 complement the ramped surfaces 132C of the endplates 102, 104, for example, with a sliding dovetail type configuration. It will be appreciated that the female/male configurations may be reversed or may include other suitable ramp interactions, sliding features, or mating components to provide expansion. When the rear and/or mid-ramps 110, 112 are translated, the ramped surfaces 188 of the front driving ramp 114 slide against the ramped surfaces 132C of the endplates 102, 104, thereby increasing or decreasing the height of the front end 108 of the implant 100 depending on the movement of the other ramps 110, 112.

The front ramp 114 defines a bore 192 between front and rear faces 184, 186 of the driving ramp 114, which is configured for receiving the drive screw actuator 116 therethrough. The bore 192 may be non-threaded to interface with a non-threaded portion 216 of the drive screw actuator 116. The non-threaded through bore 192 is laterally offset such that the axis of the non-threaded through bore 192 aligns with the lateral offset axis of the dual actuator drive assembly when assembled. The bore 192 may define a circumferential or annular groove 194 configured to receive a securing ring 244, thereby securing the actuator screw 116 to the front driving ramp 114 while still permitting rotation of the actuator screw 116 relative to the front driving ramp 114.

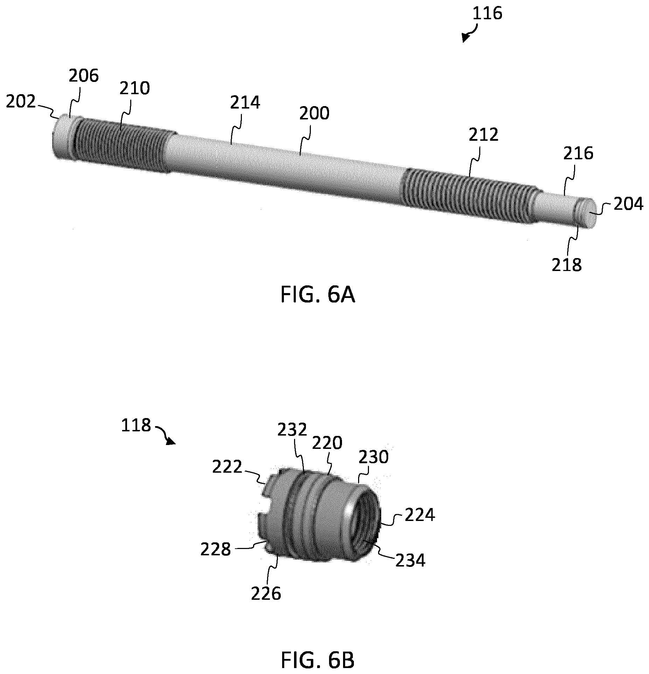

As best seen in FIG. 6A, the central drive screw actuator 116 may include a shaft 200 extending from a proximal end 202 to a distal end 204. The proximal end 202 may include an enlarged head 206 defining a drive recess 208 configured to be engaged by a driving instrument to rotate the actuator screw 116. The drive recess 208 may be a hexalobe, slot, cross, or other suitable shape that may engage with a tool or device having a corresponding tip. The shaft 200 includes a first threaded portion 210, a second threaded portion 212, a first non-threaded portion 214 between the threaded portions 210, 212, and a second non-threaded portion 216 toward the distal end 204. The first threaded portion 210 may include external threads configured to mate with the actuator nut 118, which is connected to the rear driving ramp 110. The second threaded portion 212 may have external threads configured to mate with the mid-ramp 112. The first and second threaded portions 210, 212 may have different attributes including outer diameters, handedness, thread form, thread angle, lead, pitch, etc. For example, the second threaded portion 212 may have a reduced diameter relative to the first threaded portion 210, and the first threaded portion 210 may have a finer thread pitch or more leads than the second thread portion 212. The second non-threaded portion 216 may have a reduced diameter relative to the remainder of the shaft 200. The second non-threaded portion 216 is configured to receive the front driving ramp 114. The second non-threaded portion 216 may define a circumferential or annular groove 218 near the distal end 204, which is configured to receive a retaining clip 246, thereby securing the driving screw 116 to the front driving ramp 114 while still permitting rotation of the driving screw 116 relative to the front driving ramp 114.

As best seen in FIG. 6B, the actuator nut 118 may include a cylindrical body 220 extending from a proximal end 222 to a distal end 224. The cylindrical body 220 may have an enlarged head 226 defining a second instrument recess 228 configured to be engaged by a second driving instrument to rotate the actuator nut 118. The instrument recess 228 may include a notched head or other suitable shape that may engage with a tool or device having a corresponding tip to thereby rotate the actuator nut 118. The actuator nut 118 may have a neck 230 with a reduced diameter which engages with the pronounced interior rim 153 of the rear driving ramp 110 when fitted in the bore 152 of the rear driving ramp 110. The enlarged head 226 may define a circumferential or annular groove 232 configured to receive the retaining clip 240, thereby securing the actuator nut 118 to the rear driving ramp 110 while still permitting rotation of the actuator nut 118 relative to the rear driving ramp 110. The actuator nut 118 defines a through bore 234 between the proximal and distal ends 222, 224. The through bore 234 may be at least partially threaded to engage the threaded proximal portion 210 of the actuator screw 116. For example, the through bore 234 may be internally threaded along the neck portion 230 of the actuator nut 118.

To assemble the implant 100, the top and bottom endplates 102, 104 may be attached to the drive assembly including the dual actuators 116, 118 and attached internal driving ramps 110, 112, 114. First, the dual actuators 116, 118 may be combined and fitted into the rear driving ramp 110. The proximal end 202 of the actuator screw 116 may be positioned through the distal end 224 of the through bore 234 of the actuator nut 118. The actuator nut 118 may be threaded onto the first threaded portion 210 of the actuator drive screw 116. The proximal end 202 of the actuator drive screw 116 with attached actuator nut 118 may be positioned through the bore 152 of the rear driving ramp 110. The assembly may seat into the rear driving ramp 110 with the pronounced interior rim 153 of the rear driving ramp 110 grasping the neck 230 of the actuator nut 118. The assembly may be secured to the rear driving ramp 110, for example, via one or more securing or retaining clips 240, 242. The retaining clips 240, 242 may include c-shaped clips or partial rings with a central opening. The first retaining clip 240 may fit into the annular groove 232 along the outside of the actuator nut 118 and a corresponding interior annular groove 158 within bore 152 of the rear driving ramp 110. The second retaining clip 242 may fit beneath the first retaining clip 242 around the head 226 of the actuator nut 118. The second retaining clip 242 may also rest in the interior annular groove 158 within bore 152 of the rear driving ramp 110. Although c-shaped retaining clips are shown, it will be appreciated that any suitable mechanism may be used to secure the actuator assembly to the rear driving ramp 110 while still permitting independent rotation of both actuators 116, 118.

Next, the other driving ramps 112, 114 may be added to the dual actuator assembly. The central driving ramp 112 may be added to the actuator driving screw 116. The distal end 204 of the actuator screw 116 may be positioned through the proximal end 166 of the threaded bore 172 of the mid-ramp 112. The mid-ramp 112 may be threaded onto the second threaded portion 212 of the actuator drive screw 116. Next, the front driving ramp 114 may be added to the actuator drive screw 116. The distal end 204 of the actuator screw 116 may be positioned through the proximal end 186 of the non-threaded bore 192 of the front driving ramp 114. The front driving ramp 114 may be secured, for example, via one or more securing or retaining rings 244, 246. The first retaining ring 244 may include a full ring with a central opening. The first retaining ring 244 may fit around the non-threaded portion 216 of the actuator screw 116 and into the annular groove 194 within the front driving ramp 114. The retaining ring 244 may also act as a friction ring to add friction or protect the actuator screw 116. The second retaining ring 246 may include a c-shaped clip or partial ring with a central opening. The second retaining ring 246 may be positioned adjacent to the front face 184 of the front driving ramp 114 and fit into the groove 218 in the distal end 204 of the actuator screw 116, thereby securing the front driving ramp 114 to the actuator screw 116.

Lastly, the top and bottom endplates 102, 104 may be assembled to the dual actuator drive assembly. The top and bottom endplates 102, 104 may be assembled to the rear, mid, and front driving ramps 110, 112, 114. In particular, the ramped surfaces 148 of the rear ramp 110 slidably mate with the rear ramped surfaces 132A of the top and bottom endplates 102, 104, the ramped surfaces 168 of the mid-ramp 112 slidably mate with the mid ramped surfaces 132B of the top and bottom endplates 102, 104, and the ramped surfaces 188 of the front ramp 114 slidably mate with the front ramped surfaces 132C of the top and bottom endplates 102, 104.

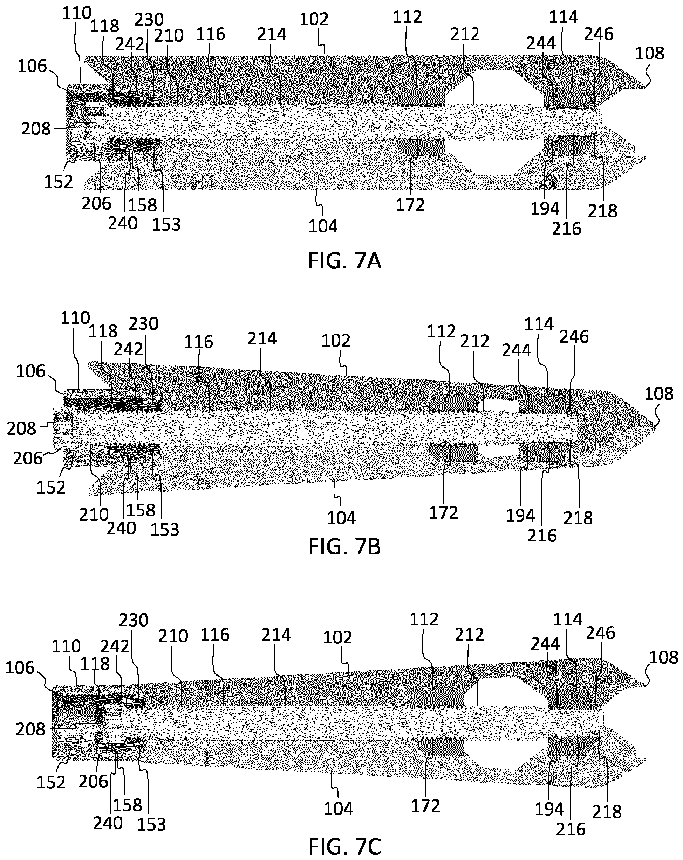

As best seen in FIGS. 7A-7C, as one or more of the driving ramp 110, 112, 114 moves, the ramped surfaces 148, 168, 188 push against the corresponding ramped surfaces 132 of the top and bottom endplates 102, 104. In this manner, the individual driving ramps 110, 112, 114 control the rate and angle of expansion of the upper and lower endplates 102, 104. FIG. 7A shows one example of the implant 100 with parallel expansion of the endplates 102, 104. For example, when both the actuator drive screw 116 and actuator nut 118 are rotated at the same time, the rear ramp 110 and mid-ramp 112 slide together in parallel, thereby moving the endplates 102, 104 in parallel. It will further be appreciated when both actuators 116, 118 are rotated simultaneously at different rates and/or in different directions, the implant 100 can be expanded in a coordinated yet varied manner at both ends 106, 108, allowing for a customized fit and coronal correction.

FIG. 7B shows one example of the implant 100 with ipsilateral height expansion. When only the actuator nut 118 is rotated, the rear ramp 110 may be translated forward along the drive screw 116 such that the rear height increases and the front height decreases. The actuator drive screw 116 may also be rotated to push the mid-ramp toward the front ramp 114 and away from the rear ramp 110, thereby creating the maximum amount of contralateral angle bias. FIG. 7C shows one example of the implant 100 with contralateral height expansion. When the actuator nut 118 remains stationary (and positioned toward the rear end 106) and only the actuator drive screw 116 is rotated, the mid-ramp 112 may be drawn back toward the rear ramp 110 such that the front height increases and the rear height decreases, thereby creating an ipsilateral angle bias. Depending on the direction and amount of rotation of the actuator(s) 116, 118, the rear and mid-ramps 110, 112 are permitted to travel along the length of the respective threaded portions 210, 212 of the drive screw 116 to allow for precise and controlled adjustments of the height and coronal angle. It will be appreciated that the movement of the driving ramps 110, 112, 114 and resulting expansion may be operated by the concentric actuators 116, 118 with any suitable configurations and mechanisms to obtain the desired expansion and angled bias for coronal correction.

In one embodiment, the endplates 102, 104 or implant as a whole may have a reverse taper, and after insertion, the implant 100 may be adjusted to a neutral or opposite tilt to correct the coronal deformity. In this design, the endplates 102, 104 or body of the implant 100 may be shaped with a reverse taper to match the obliquity or angular disposition of the insertion site, for example, to allow for easier insertion into the disc space. After insertion, the endplates 102, 104 may be tilted to neutral or beyond, such that the taper is reversed to change the angle of the endplates 102, 104 relative to one another. In this manner, the implant 100 can be manipulated to shift from its initial oblique alignment to a more neutral or adjusted angular position to achieve optimal spinal alignment and stability.

During use, the implant 100 may enter the disc space in a collapsed orientation, and may then be expanded to achieve height correction and coronal correction to a desired coronal angle. The implant 100 may be installed laterally or anteriorly, for example, using an inserter instrument such that the implant 100 is aligned with an ipsilateral side and a contralateral side of the disc space. After installation, the implant height and angle may be adjusted by turning one or both of the concentric actuators 116, 118 for achieving the desired alignment and coronal angle, thereby correcting the coronal deformity. The asymmetric coronal expansion provides for independent ipsilateral and contralateral height expansion for a customized patient fit. The angle bias may be used to match the obliquity of the vertebral endplates and/or may be used to correct the spinal deformity, such as vertebral wedging or scoliosis, thereby correcting the bend in the coronal plane, realigning the spine, and restoring the biomechanical balance of the spine.

Turning now to FIGS. 8A-8B, examples of expandable coronal implants 300, 300A with pin and slot actuators are shown. In these embodiments, the mating ramped surfaces are replaced with pins that ride along angled slots in the sides of the implant to control implant expansion. The expandable implants 300, 300A are shown with a portion of the upper endplate 302 omitted for clarity. In FIG. 8A, the expandable implant 300 includes an angled slot 314 to allow for angulation in one direction. In FIG. 8B, an alternative expandable implant 300A includes a slot 314 with multiple angles to allow for multiple phases of endplate angulation.

With further reference to FIG. 8A, the implant 300 includes top and bottom endplates 302, 304, which are configured to engage with adjacent vertebrae. The implant 300 extends along a central longitudinal axis between rear end 306 (or ipsilateral side) configured to connect with an insertion instrument and front end 308 (or contralateral side) configured to be inserted first into the disc space. The implant 300 may define a frame 310, for example, having a rectangular shape. A pair of rails 312 may extend along each side of the frame 310. Each rail 312 may define an angled slot 314. The angled slot 314 may have a given slope, for example, increasing or decreasing along the length of the rail 312. The implant 300 includes a driving ramp 316 with a pin 318 extending therefrom. The pin 318 may include a round peg or dowel sized and dimensioned to fit within the angled slots 314. The rails 312 may be pinned to one end of the frame 310, for example, with a hinge or pivot pin 320. The driving ramp 316 may be translated, for example by a threaded driving screw (e.g., similar to implant 100) or other suitable mechanism. As the pins 318 move within the angled slots 314, the pins 318 push against the sides of the slots 314 causing the endplates 302, 304 to move outward or change angulation. In this embodiment, the bottom endplate 304 expands to increase the ipsilateral height. It will be appreciated that the slots 314 may be reconfigured for contralateral expansion. The location and angle of the slots 314 dictate how the forces applied by the pins 318 are translated into movement of the endplate(s) 302, 304, allowing for precise control over the expansion.

In the alternative embodiment shown in FIG. 8B, each angled slot 314 is divided into two distinct angled portions 322, 324 with different directions of slope. The first angled slot portion 322 may include decreasing slope while the second angled slot portion 324 includes increasing slope (or vice versa). The two angled portions 322, 324 meet in the middle, for example, at a low point to provide the transition between two types of expansion. In this embodiment, the rails 312 may be secured at both ends of the frame 310, for example, with pins 326, which travel along respective vertical slots in the rails 312. The dual-slope configuration allows for adjustments to be made in multiple directions depending on which part of the slot 314 the pin 318 is engaged with. By moving the pin 318 forward or backward within the slot 314, the pin 318 can engage with either the upward sloping portion or the downward sloping portion causing different angulation of the endplates 302, 304 (e.g., ipsilateral or contralateral angulation) of the implant 300A.

Turning now to FIGS. 9A-9B, an alternative embodiment of an expandable coronal implant 400 with two separate pin in slot actuators is shown. In this embodiment, the pins ride along angled surfaces but are controlled by two separate internal drive screws to control implant expansion and angulation. With further reference to FIG. 9A, the implant 400 includes top and bottom endplates 402, 404, which are configured to engage with adjacent vertebrae. The implant 400 extends along a central longitudinal axis between rear end 406 (or ipsilateral side) configured to connect with an insertion instrument and front end 408 (or contralateral side) configured to be inserted first into the disc space. The implant 400 may include a pair of driving ramps 410, 412 each with one or more pins 418 extending therefrom. The pins 418 may include a round peg or dowel sized and dimensioned to slide against ramped surfaces 420 along the top and bottom endplates 402, 404.

The ramped surfaces 420 along the top and bottom endplates 402, 404 may be divided into different areas with different directions of slope. Similar to the dual-slope slots 314, a first angled surface may include decreasing slope while the second angled surface may include increasing slope (or vice versa). The two angled surfaces may meet in the middle, for example, at a high point (e.g., for the top endplate 402) or a low point (e.g., for the bottom endplate 404), but each driving ramp 410 may only follow one side of the sloped surfaces. For example, the first driving ramp 410 may slide between a first area of decreasing slope and the second driving ramp 412 may slide between a second area of increasing slope, in the opposite direction, for the ramped surfaces 420 (although each may independently slide up or down the sloped surfaces). It will be appreciated that the dual-slope and sliding arrangement may be reconfigured or modified for the desired expansion.

Each driving ramp 410, 412 may be independently driven via separate actuator drive screws 422, 424. Similar to actuator drive screw 116, the screws 422, 424 may have a threaded portion which threadedly mates with the respective driving ramps 410, 412. As each actuator screw 422, 424 is rotated, the driving ramps 410, 412 are independently translated forward or backward such that the pins 418 travel along the ramped surfaces 420 of the top and bottom endplates 402, 404. In this manner, each end 406, 408 of the implant 400 may be independently controlled with different driving ramps 410, 412 engaging with different areas of the ramped surfaces 420 to allow for independent raising of near or far side of the implant 400. By independently moving the driving ramps 410, 412 forward or backward along the ramped surfaces 420, the implant 400 may be expanded with the desired ipsilateral or contralateral angulation to correct the coronal imbalance.

Turning now to FIGS. 10A-10B, one example of a coronal expandable implant 500 is shown to provide a coronal target approach when the implant 500 is installed laterally into the disc space. Expandable implant 500 includes top and bottom endplates 502, 504, rear end 506 (or ipsilateral side), and front end 508 (or contralateral side). The implant 500 may include a housing or body 510 positioned between the endplates 502, 504, a nose 512, and a single drive screw 514. The endplates 502, 504 may have two main pairs of ramps that allow for expansion: two back ramps that mate with the two ramps of the body 510, and two front ramps that mate with the ramps of the nose 512. The drive screw 514 provides a threaded mechanism for expanding and contracting the expandable fusion device 500. Further details on this type of expandable implant may be found in U.S. Patent No. 11,191,650, which is incorporated by reference herein in its entirety for all purposes. When installed laterally into the disc space, the front end 508 of the implant 500 may be expanded, for example, to increase the contralateral height and provide ipsilateral angle bias to provide a coronal target approach.

Unlike static implants with a coronal taper, the implants described herein may allow for adjustable coronal angle and expansion height. The implants may be provided in a small footprint suitable for minimally invasive procedures where the implant is inserted in a collapsed configuration and then expanded to restore disc spacing, fix coronal deformities, and enhance spinal stability. The implants may be particularly suited for MIS lateral implantation to address disc height restoration as well as asymmetric coronal expansion. The implants may be configured for contralateral and/or ipsilateral coronal angulation depending on the deformity and correction desired. The implants may offer the precise adjustment needed in surgeries for patients having a coronal imbalance and/or vertebral wedging, thereby restoring the disc space and providing coronal correction. In some cases, the implants may provide for independently controlled dual expansion, which allows for independent ipsilateral and contralateral height expansion to address asymmetries and misalignments, to thereby achieve optimal alignment and stabilization of the spine.

Although the invention has been described in detail and with reference to specific embodiments, it will be apparent to one skilled in the art that various changes and modifications can be made without departing from the spirit and scope of the invention. Thus, it is intended that the invention covers the modifications and variations of this invention provided they come within the scope of the appended claims and their equivalents. It is expressly intended, for example, that all components of the various devices disclosed above may be combined or modified in any suitable configuration.

The invention could be inter alia defined by the following Examples:
1.An expandable coronal implant comprising: a top endplate and a bottom endplate configured to engage adjacent vertebrae; a dual actuator assembly including a rotatable drive screw actuator having a shaft and a rotatable actuator nut concentric to the drive screw actuator; and a plurality of driving ramps including a front ramp, a mid-ramp, and a rear ramp positioned along the shaft of the drive screw actuator and engaged with the top and bottom endplates via complementary ramped surfaces, wherein when inserted in a disc space, the implant has an ipsilateral side and a contralateral side, and wherein rotation of the drive screw actuator and/or the actuator nut causes movement of one or more of the driving ramps, thereby causing independent expansion in height of the contralateral and/or ipsilateral sides of the implant to correct a coronal deformity.
2. The expandable implant of Example 1, wherein the dual actuator assembly is laterally offset relative to a central longitudinal axis of the implant, and the front ramp, mid-ramp, and rear ramp define through bores along the offset axis to receive the shaft of the drive screw actuator.
3. The expandable implant of Example 1, wherein the rear ramp defines female ramped grooves forming a male triangular area with a vertex pointing toward the front of the implant, the mid-ramp defines female ramped grooves forming a male triangular area with a vertex pointing toward the rear of the implant, and the front ramp defines female ramped grooves forming a male triangular area with a vertex pointing toward the front of the implant.
4. The expandable implant of Example 1, wherein the shaft of the drive screw actuator includes a first threaded portion, a second threaded portion, a first non-threaded portion separating the first threaded portion from the second threaded portion, and a second non-threaded portion toward a distal end of the shaft.
5. The expandable implant of Example 4, wherein the rear ramp is positioned on the actuator nut, which is positioned on the first threaded portion of the drive screw actuator, the mid-ramp is positioned on the second threaded portion of the drive screw actuator, and the front ramp is positioned on the second non-threaded portion of the drive screw actuator.
6. The expandable implant of Example 5, wherein the actuator nut includes a cylindrical body having an enlarged head defining an instrument recess and a neck with a reduced diameter defining an internal threaded portion, which engages with the first threaded portion of the shaft of the drive screw actuator.
7. The expandable implant of Example 5, wherein the actuator nut is secured to the rear ramp with a first retaining clip fit into an annular groove along an outside of the actuator nut and a corresponding interior annular groove along an interior of the rear ramp.
8. The expandable implant of Example 7, wherein the actuator nut is secured to the rear ramp with a second retaining clip fit beneath the first retaining clip around the head of the actuator nut and resting in the interior annual groove along the interior of the rear ramp.
9. An expandable coronal implant comprising: a top endplate and a bottom endplate configured to engage adjacent vertebrae, wherein the top and bottom endplates are angled to provide an initial reverse taper configured to match an angle of a disc space upon insertion; a dual actuator assembly including a rotatable drive screw actuator having a shaft and a rotatable actuator nut concentric to the drive screw actuator; and a plurality of driving ramps including a front ramp, a mid-ramp, and a rear ramp positioned along the shaft of the drive screw actuator and engaged with the top and bottom endplates via complementary ramped surfaces, wherein after insertion into the disc space, the implant is expanded by rotating the drive screw actuator and/or the actuator nut to cause movement of one or more of the driving ramps, thereby causing the endplates to expand to provide a neutral position or angle opposite to the initial reverse taper to correct a coronal deformity.
10. The expandable implant of Example 9, wherein the implant has an ipsilateral side and a contralateral side in the disc space, and the initial reverse taper has an asymmetric taper such that the contralateral side is greater in height than the ipsilateral side.
11. The expandable implant of Example 10, wherein the implant is expandable to tilt the top and bottom endplates to the angle opposite to the initial reverse taper such that the ipsilateral side is greater in height than the contralateral side to correct the coronal deformity.
12. The expandable implant of Example 10, wherein the implant is expandable such that the top and bottom endplates are parallel to one another in the neutral position.
13. A method of correcting a coronal deformity comprising: inserting an implant through a lateral approach into a disc space of a spine having a coronal deformity, the implant having top and bottom endplates, a dual actuator assembly including a rotatable drive screw actuator having a shaft and a rotatable actuator nut concentric to the drive screw actuator, and a plurality of driving ramps including a front ramp, a mid-ramp, and a rear ramp positioned along the shaft of the drive screw actuator and engaged with the top and bottom endplates via complementary ramped surfaces; expanding the implant to a height to restore disc spacing; and independently adjusting the height of contralateral and ipsilateral sides of the implant to provide a desired coronal angle.
14. The method of Example 13, wherein the drive screw actuator and actuator nut are rotated simultaneously to move the rear and mid-ramp together in parallel, thereby moving the top and bottom endplates in parallel.
15. The method of Example 13, wherein only the actuator nut is rotated, and the rear ramp is translated forward to increase the ipsilateral side of the implant.
16. The method of Example 13, wherein the only the drive screw actuator is rotated, and the mid-ramp is drawn backward to increase the contralateral side of the implant.
17. The method of Example 13, wherein the implant includes an initial reverse taper to match an obliquity of the disc space upon insertion.
18. The method of Example 17, wherein after insertion, the top and bottom endplates are tilted to a neutral position to achieve optimal spinal alignment.
19. The method of Example 17, wherein after insertion, the top and bottom endplates are tilted to angle the top and bottom endplates opposite to the initial reverse taper, thereby providing an adjusted angular position for optimal correction.
20. The method of Example 13 further comprising correcting any compensatory curves above the coronal deformity because such a compensation is no longer necessary.

## Claims

1. An expandable coronal implant comprising:
a top endplate and a bottom endplate configured to engage adjacent vertebrae;
a dual actuator assembly including a rotatable drive screw actuator having a shaft and a rotatable actuator nut concentric to the drive screw actuator; and
a plurality of driving ramps including a front ramp, a mid-ramp, and a rear ramp positioned along the shaft of the drive screw actuator and engaged with the top and bottom endplates via complementary ramped surfaces,
wherein when inserted in a disc space, the implant has an ipsilateral side and a contralateral side, and wherein rotation of the drive screw actuator and/or the actuator nut causes movement of one or more of the driving ramps, thereby causing independent expansion in height of the contralateral and/or ipsilateral sides of the implant to correct a coronal deformity.

2. The expandable implant of claim 1, wherein the dual actuator assembly is laterally offset relative to a central longitudinal axis of the implant, and the front ramp, mid-ramp, and rear ramp define through bores along the offset axis to receive the shaft of the drive screw actuator.

3. The expandable implant of claim 1 or 2, wherein the rear ramp defines female ramped grooves forming a male triangular area with a vertex pointing toward the front of the implant, the mid-ramp defines female ramped grooves forming a male triangular area with a vertex pointing toward the rear of the implant, and the front ramp defines female ramped grooves forming a male triangular area with a vertex pointing toward the front of the implant.

4. The expandable implant of any one of the preceding claims , wherein the shaft of the drive screw actuator includes a first threaded portion, a second threaded portion, a first non-threaded portion separating the first threaded portion from the second threaded portion, and a second non-threaded portion toward a distal end of the shaft.

5. The expandable implant of claim 4, wherein the rear ramp is positioned on the actuator nut, which is positioned on the first threaded portion of the drive screw actuator, the mid-ramp is positioned on the second threaded portion of the drive screw actuator, and the front ramp is positioned on the second non-threaded portion of the drive screw actuator.

6. The expandable implant of claim 5, wherein the actuator nut includes a cylindrical body having an enlarged head defining an instrument recess and a neck with a reduced diameter defining an internal threaded portion, which engages with the first threaded portion of the shaft of the drive screw actuator.

7. The expandable implant of claim 5, wherein the actuator nut is secured to the rear ramp with a first retaining clip fit into an annular groove along an outside of the actuator nut and a corresponding interior annular groove along an interior of the rear ramp.

8. The expandable implant of claim 7, wherein the actuator nut is secured to the rear ramp with a second retaining clip fit beneath the first retaining clip around the head of the actuator nut and resting in the interior annual groove along the interior of the rear ramp.

9. The expandable implant of any one of the preceding claims
wherein the top and bottom endplates are angled to provide an initial reverse taper configured to match an angle of a disc space upon insertion;
wherein after insertion into the disc space, the implant is expanded by rotating the drive screw actuator and/or the actuator nut to cause movement of one or more of the driving ramps, thereby causing the endplates to expand to provide a neutral position or angle opposite to the initial reverse taper to correct a coronal deformity.

10. The expandable implant of any one of the preceding claims, wherein the implant has an ipsilateral side and a contralateral side in the disc space, and the initial reverse taper has an asymmetric taper such that the contralateral side is greater in height than the ipsilateral side.

11. The expandable implant of claim 10, wherein the implant is expandable to tilt the top and bottom endplates to the angle opposite to the initial reverse taper such that the ipsilateral side is greater in height than the contralateral side to correct the coronal deformity.

12. The expandable implant of claim 10, wherein the implant is expandable such that the top and bottom endplates are parallel to one another in the neutral position.
